# EUROPÄISCHE PATENTSCHRIFT

(11) **EP 2 340 015 B1**
(45) Veröffentlichungstag und Bekanntmachung des Hinweises auf die Patenterteilung: **16.12.2015**
(21) Anmeldenummer: 09737355.9
(22) Anmeldetag: 14.10.2009
(51) Int. Cl.: A61K 9/00, A61K 9/70

(54) **STEUERBARES THERAPEUTISCHES SYSTEM**
CONTROLLABLE THERAPEUTIC SYSTEM
SYSTÈME THÉRAPEUTIQUE APTE À ÊTRE COMMANDÉ

(30) Priorität: 30.10.2008 DE 102008053889
(43) Veröffentlichungstag der Anmeldung: 06.07.2011
(73) Patentinhaber: LTS LOHMANN Therapie-Systeme AG, 56626 Andernach (DE)
(72) Erfinder: MAIER, Stephan, 51371 Leverkusen (DE)
(74) Vertreter: Siemund, Volker
(86) Internationale Anmeldenummer: PCT/EP2009/007362
(87) Internationale Veröffentlichungsnummer: WO 2010/049062

(56) Entgegenhaltungen:
- EP-A- 1 658 861
- WO-A-02/100386

## Beschreibung

Die Erfindung betrifft ein therapeutisches System, das laminatförmig aufgebaut ist und das zumindest eine adhäsive Laminatschicht umfasst sowie eine Therapievorrichtung mit einem derartigen therapeutischen System.

Laminatförmige therapeutische Systeme sind z.B. Pflaster, die bei der Anwendung auf die Haut des Patienten aufgeklebt sind. Diese Pflaster können pharmazeutische Wirkstoffe enthalten oder wirkstofffrei ausgeführt sein. Bei wirkstoffhaltigen therapeutischen Systemen dringen die Wirkstoffe bei der Anwendung in oder durch die Haut des Patienten. Pflaster, bei denen der Wirkstoff durch die Haut dringt, werden beispielsweise als transdermale therapeutische Systeme bezeichnet.

Bei der Anwendung therapeutischer Systeme beeinträchtigt die Temperatur des therapeutischen Systems und/oder die Temperatur des aufnehmenden Körpers die Wirkung der Therapie. Beispielsweise führt eine Temperaturerhöhung des Systems zu erhöhten Diffusionskoeffizienten der Wirkstoffe in den Zubereitungen und somit zu einer verbesserten Wirkstoffabgabe. Daneben kann durch eine Temperaturerhöhung des Systems die Löslichkeit von Wirkstoffen der Zubereitung verbessert werden. So kann in Formulierungen, die einen Anteil ungelösten Wirkstoffs aufweisen, durch eine Temperaturerhöhung zusätzlicher Wirkstoff aufgelöst werden. Die Konzentration des Wirkstoffs in der Formulierung wird damit erhöht. Dies erhöht die thermodynamische Kraft des Systems und damit das Bestreben, den Wirkstoff an die Haut abzugeben.

Eine Temperaturerhöhung der Hautoberfläche kann ebenfalls die Wirkstoffaufnahme in und durch die Haut beschleunigen. Mit beiden Maßnahmen kann eine verstärkte Wirkung des Arzneimittels erreicht werden.

Aus der WO 02/100386 A1 ist eine Therapievorrichtung mit einem therapeutischen System bekannt, dessen Temperatur verändert werden kann. Die Wärmeübertragung auf das therapeutische System oder auf die Haut erfolgt mittels Kontakt- und/oder Strahlungswärme. Bei Wärmeübertragung durch Kontakt ist ein vollflächiger, enger und lückenloser Kontakt zwischen der Wärmequelle und dem therapeutischen System erforderlich. Unebenheiten oder Unterbrechungen der Kontaktfläche können zu Störungen der Wärmeübertragung führen.

Bei einer Wärmeübertragung durch Wärmestrahlung, z.B. durch Infrarotstrahlung, hängt die übertragene Wärmemenge vom Abstand der Strahlungsquelle zum therapeutischen System ab. Zudem hat der Winkel, in dem die Strahlungsquelle zur Systemoberfläche steht, einen Einfluss auf die Güte der Wärmeübertragung. Reflexionseffekte und variierende Abstände aufgrund von Unebenheiten, Falten und Körperrundungen führen zu ungleichmäßiger und somit schlecht kontrollierbarer Wärmezufuhr.

Der vorliegenden Erfindung liegt daher die Problemstellung zugrunde, ein von Kontaktflächen unabhängiges, durch Temperaturveränderung steuerbares therapeutisches System und eine Therapievorrichtung mit einem derartigen steuerbaren therapeutischen System zu entwickeln.

Diese Problemstellung wird mit den Merkmalen des Hauptanspruches gelöst. Dazu enthält zumindest eine Laminatschicht oder eine und/oder eine zwischen zwei Laminatschichten angeordnete Zwischenschicht des therapeutischen Systems superparamagnetische Nanopartikel. Die Therapievorrichtung mit diesem therapeutischen System umfasst mindestens ein lösbar mit dem therapeutischen System verbundenes elektrisch zeitlich veränderlich ansteuerbares System. Das elektrisch ansteuerbare System weist einen frequenzabhängigen elektrischen Widerstand auf. Beim Betrieb der Therapievorrichtung erzeugt das elektrisch ansteuerbare System ein die Ausrichtung und die Stärke zeitlich veränderndes, die superparamagnetischen Nanopartikel durchdringendes elektrisches oder magnetisches Feld.

Weitere Einzelheiten der Erfindung ergeben sich aus den Unteransprüchen und der nachfolgenden Beschreibung schematisch dargestellter Ausführungsformen.
- Figur 1:: Therapievorrichtung bei der Anwendung;
- Figur 2:: Teillängsschnitt durch Figur 1;
- Figur 3:: Detail der Therapievorrichtung aus Figur 1;
- Figur 4:: Teillängsschnitt durch eine Therapievorrichtung mit einem Therapiesystem mit integrierter Induktionsspule und mit einem abnehmbaren Induktionssystem;
- Figur 5:: Therapievorrichtung mit zylindrischer Spule;
- Figur 6:: Induktionssystem;
- Figur 7:: Therapievorrichtung mit einem kapazitiven System;
- Figur 8:: Draufsicht eines kapazitiven Systems.

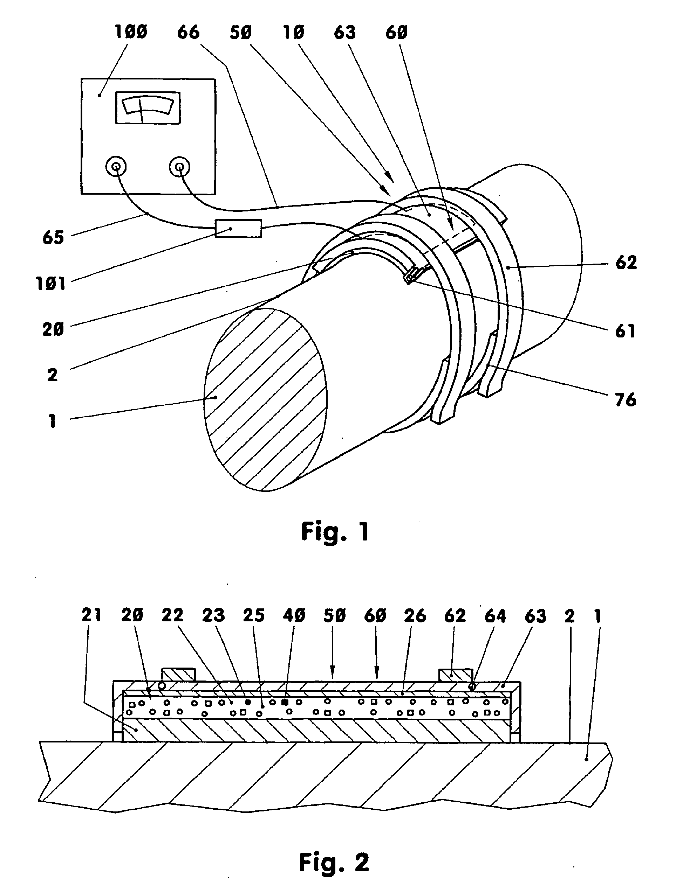

Die Figuren 1 und 2 zeigen eine Therapievorrichtung (10) bei der Anwendung, z.B. an einem Arm (1) eines Patienten in einer dimetrischen Ansicht und in einem Längsschnitt. Die Therapievorrichtung (10) umfasst in diesem Ausführungsbeispiel ein therapeutisches System (20) und ein elektrisch zeitlich veränderlich ansteuerbares System (50). Das elektrisch ansteuerbare System (50) ist z.B. ein Induktionssystem (60). Dieses ist lösbar kraft- und/oder formschlüssig mit dem therapeutischen System (20) verbunden.

Das therapeutische System (20) ist laminatförmig aus z.B. mehreren zueinander kongruenten Laminatschichten (21, 22) aufgebaut. Im Ausführungsbeispiel umfasst es beispielsweise eine der Haut (2) des Patienten zugewandte adhäsive Laminatschicht (21) und eine zu dieser Laminatschicht (21) kongruente wirkstoffhaltige obere Laminatschicht (22). Auf der der Haut (2) des Patienten abgewandten Seite ist das therapeutische System (20) z.B. mittels einer das therapeutische System (20) schützenden Deckfolie (26) abgedeckt.

Gegebenenfalls kann das therapeutische System (20) nur eine einzige Laminatschicht umfassen. Diese ist dann sowohl die adhäsive, selbst haftklebende Schicht als auch die wirkstoffhaltige Schicht.

Die adhäsive, selbst haftklebende Laminatschicht (21) ist beispielsweise eine lipophile, halbfeste Klebemasse. Sie ist z.B. druckempfindlich. Das bedeutet, dass die Haftwirkung der Laminatschicht (21) auf der Haut (2) unter der Einwirkung eines äußeren Drucks verstärkt wird.

In der wirkstoffhaltigen Laminatschicht (22) dieses transdermalen therapeutischen Systems (20) sind die Wirkstoffe (23) beispielsweise in eine Verbundmasse (25) gelartiger Konsistenz eingelagert.

Die pharmazeutische Wirkstoffzubereitung (23) kann auch in die adhäsive Laminatschicht (21) eingebettet sein. Auch ist ein transdermales therapeutisches System (20) mit mehreren z.B. übereinander angeordneten Schichten (22) denkbar. Gegebenenfalls kann das therapeutische System (20) ohne eine wirkstoffhaltige Schicht (22) ausgeführt sein. Beispielsweise dient es dann als wirkstofffreies Pflaster zur Schmerzlinderung durch Wärmetherapie.

Das transdermale therapeutische System (20) hat in diesem Ausführungsbeispiel eine quadratische Grundfläche mit einer Kantenlänge von 70 Millimetern. Die Grundfläche kann aber auch rechteckig, rund, etc. sein. Die Dicke des Systems (20) beträgt hier beispielsweise zwischen 50 und 300 Mikrometern.

Beispielsweise in der wirkstoffhaltigen Schicht (22) sind superparamagnetische Nanopartikel (40) eingelagert. Dies sind z.B. ferrimagnetische Partikel aus Magnetit (Fe₃O₄) oder Maghemit (γ-Fe₂O₃). Die Partikel können aber auch aus einem Werkstoff der oxidkeramischen Gruppe der Ferrite bestehen. Die Korngröße der einzelnen Kristalle beträgt zwischen fünf Nanometern und dreißig Nanometern. Gegebenenfalls können die superparamagnetischen Nanopartikel (40) auch stab- oder drahtförmig ausgebildet sein. Bei einem einschichtigen transdermalen therapeutischen System (20) sind in dieser Schicht auch die superparamagnetischen Nanopartikel (40) eingelagert.

Diese Kristalle sind zunächst magnetisch neutral. In einem äußeren Magnetfeld werden sie jedoch magnetisch ausgerichtet. Aufgrund ihrer geringen Größe haben die Kristalle meist nur einen sogenannten Weiss 'schen Bezirk gleicher magnetischer Orientierung. Die Ausrichtung dieses Bezirks folgt den Magnetfeldlinien des äußeren Magnetfelds. Hierbei entsteht keine Hysterese und somit auch keine Remanenz. Beim Abschalten des äußeren Magnetfelds ist das Kristall wieder magnetisch neutral.

Die Konzentration der superparamagnetische Nanopartikel (40) in der jeweiligen Laminatschicht (21, 22) kann zwischen 0,5 Massenprozent und 25 Massenprozent betragen. Beispielsweise beträgt sie zwischen einem und 15 Massenprozent. Im Ausführungsbeispiel liegt die Konzentration der superparamagnetischen Nanopartikel (40) zwischen zwei und zehn Massenprozent der oberen Laminatschicht (22). Hierbei kann die Konzentration der superparamagnetischen Nanopartikel (40) gleichmäßig im gesamten therapeutischen System (20) sein oder die einzelnen Laminatschichten (21, 22) weisen ungleichmäßige Konzentrationen auf.

Die Deckfolie (26) ist beispielsweise mechanisch reißfest sowie wirkstoff-, aroma- und feuchtigkeitsundurchlässig. Das transdermale therapeutische System (20) ist somit auch bei längerem Einsatz vor Beschädigungen und ungewollten Wirkstoffverlusten geschützt.

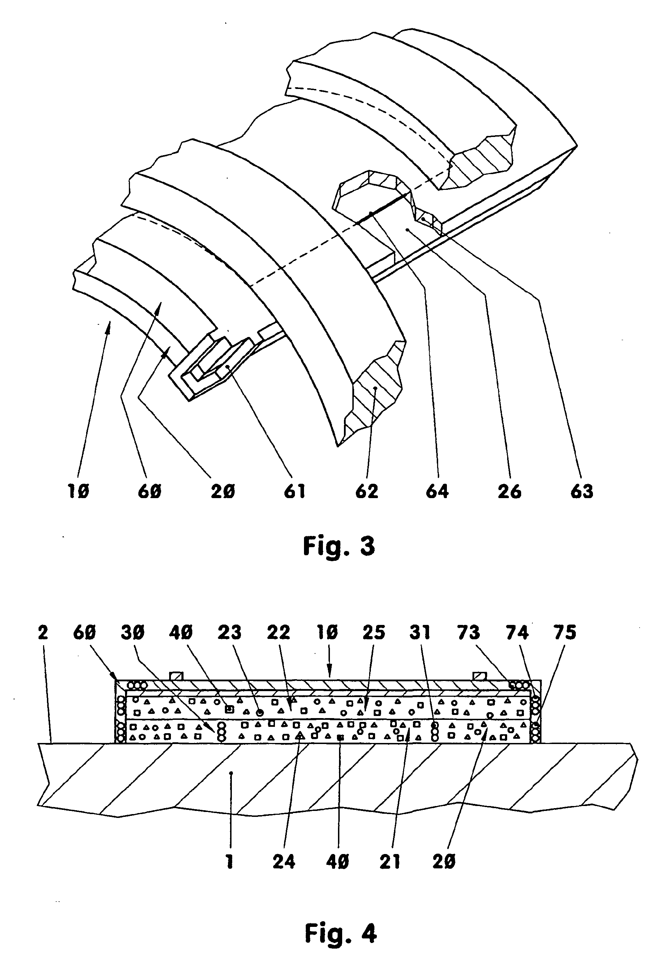

Das Induktionssystem (60) liegt auf der Deckfolie (26) des therapeutischen Systems (20) auf und umgreift in der Darstellung der Figur 1 den Arm (1) des Patienten mit zwei Umgriffsbändern (62). Diese sind jeweils mittels eines lösbaren Haftverschlusses (76) verschlossen. Die Lage des Induktionssystems (60) relativ zum therapeutischen System (20) ist beispielsweise mittels zweier einander diagonal gegenüber angeordneten Umgriffsklammern (61) gesichert, vgl. Figur 3.

Im dargestellten Ausführungsbeispiel umfasst das Induktionssystem (60) z.B. einen folienartigen Spulenträger (63) und eine im Spulenträger (63) eingebettete Induktionsspule (64). Die Induktionsspule (64) ist beispielsweise als Leiterschleife mit einer Windung ausgeführt. Die Stirnfläche der Induktionsspule (64) hat z.B. einen konstanten Abstand zur Haut (2) des Patienten. Die Induktionsspule (64) kann auch mehr als eine Windung und/oder einen Spulenkern aufweisen.

Das Induktionssystem (60) ist mittels zweier elektrischer Leitungen (65, 66) über einen Vorwiderstand (101) an eine Wechselstromquelle (100) angeschlossen. Als Stromquelle kann jedoch auch eine Gleichstromquelle mit nachgeschaltetem Wechselrichter eingesetzt werden. Diese kann z.B. im Induktionssystem (60) integriert sein.

Zum Einsatz des therapeutischen Systems (20) wird dieses z.B. nach dem Abziehen einer Schutzfolie von der adhäsiven Laminatschicht (21) auf den Arm (1) eines Patienten aufgeklebt. In der Folge dringt z.B. Wirkstoff (23) aus der wirkstoffhaltigen Schicht (22) durch die adhäsive Schicht (21) hindurch in und je nach Art der Wirkstoffmoleküle auch durch die Haut (2) des Patienten. Die superparamagnetischen Nanopartikel (40) beeinflussen diesen Vorgang zunächst nicht.

Soll der Wirkstoffeintrag in bzw. der Wirkstofftransport durch die Haut (2) verstärkt werden, wird zuerst das Induktionssystem (60) am therapeutischen System (20) z.B. mittels der Umgriffsklammern (61) fixiert und am Arm (1) des Patienten mittels der Umgriffsbänder (62) lösbar befestigt. Nun wird die Stromquelle (100) beispielsweise mit konstanter Frequenz und mit einem konstanten Wert der effektiven Wechselspannung eingeschaltet. Der Effektivwert der Spannung ist der quadratische Mittelwert des zeitlich veränderlichen Spannungswerts. Der resultierende Strom, die Spannung und die Frequenz liegen unterhalb eines Grenzwerts, der für den menschlichen Körper gefährlich sein könnte oder der bei versehentlicher Berührung elektrisch leitender Teile Nebenwirkungen auslösen könnte.

Beim Betrieb des Induktionssystems (60) bewirkt der die Spule (64) durchfließende Strom den Aufbau eines magnetischen Felds um den stromdurchflossenen Leiter. Alle Dipole im Magnetfeld versuchen, sich in Richtung der Feldlinien auszurichten. Die magnetische Feldstärke steigt mit dem Strom und mit zunehmendem Verhältnis der Windungszahl zur Spulenlänge. In einem Wechselstromkreis steigen der Scheinwiderstand und die auf die Zeit bezogenen Änderungsraten des magnetischen Flusses und der magnetischen Flußdichte einer Induktionsspule (64) mit zunehmender Frequenz an.

Bei der Beaufschlagung mit Wechselstrom ändert sich das magnetische Feld mit der Zeit. Das Feld ändert sowohl seine Stärke als auch seine Richtung. Es durchdringt die superparamagnetischen Nanopartikel (40). In den fest in die Verbundmasse (25) eingebundenen superparamagnetischen Nanopartikeln (40) erfolgt hierdurch eine Veränderung der Polarisationsrichtung. Die Frequenz der Polarisationsänderung entspricht der Frequenz des von der Stromquelle (100) abgegebenen Wechselstroms. Ein Teil der hierfür von den superparamagnetischen Nanopartikeln (40) aufgenommenen Energie wird als Wärme an deren nähere Umgebung abgegeben.

Die im Ausführungsbeispiel in der wirkstoffhaltigen Laminatschicht (22) freigesetzte Wärmeenergie führt zu deren Erwärmung. Hiermit wird beispielsweise eine erhöhte Diffusion des gelösten Wirkstoffs (23) innerhalb des Systems und zur Haut erreicht. Auch kann z.B. zunächst ungelöst vorliegender Wirkstoff gelöst werden. Dadurch kann die Diffusionsrate des therapeutischen Systems (20) erhöht werden und beispielsweise der Wirkstoff (23) besser ausgenutzt werden.

Die Wärmeentwicklung und damit die Steigerung der Diffusionsrate sind abhängig von der zugeführten Energie und der magnetischen Flußdichte des Induktionssystems (60). Um die zugeführte Energie zu verändern, kann z.B. die Frequenz des Wechselstroms, die effektive Spannung, der effektive Strom oder die Einschaltdauer der Stromquelle (100) verändert werden. Beispielsweise ist die von den superparamagnetischen Nanopartikeln (40) abgegebene Energie pro Zeiteinheit umso höher, desto höher der Strom im Induktionssystem (60) ist und desto höher die Frequenz des Wechselstroms ist. Um die effektive Stromstärke in dem an die Stromquelle (100) angeschlossenen Stromkreis zu erhöhen, kann z.B. bei konstanter effektiver Spannung der Vorwiderstand und/oder der induktive Widerstand verringert werden.

Die magnetische Flußdichte des Induktionssystems kann beispielsweise mittels eines Spulenkerns vergrößert werden. Da die vektorielle Größe der magnetischen Flußdichte zu jedem Zeitpunkt von der Richtung und dem Betrag des Erregerstroms abhängig ist, ändert sich die magnetische Flußdichte mit dem angelegten Wechselstrom.

Somit kann mittels der Stromquelle (100) und mittels des Induktionssystems (60) die Wirkstoffeinbringung in bzw. der Wirkstofftransport durch die Haut (2) des Patienten gesteuert werden.

Sobald die Stromquelle abgeschaltet wird, wird die Wärmeerzeugung unterbrochen. Die Diffusionsrate des Wirkstoffs (23) nimmt ab, bis sie wieder ihren Ausgangswert erreicht hat. Das Induktionssystem (60) kann nach dem Lösen der Umgriffsbänder (62) vom therapeutischen System (20) und vom Arm (1) des Patienten abgenommen werden.

Bei einem therapeutischen System (20), dessen die Haut kontaktierende Laminatschicht (21) superparamagnetische Nanopartikel (40) enthält, kann durch die Erwärmung dieser Schicht gezielt die Haut (2) im Bereich der Kontaktzone erwärmt werden. Hierdurch wird der Wirkstofftransport in und durch die Haut (2) beschleunigt. Auch kann eine gezielte Erwärmung der Hautkontaktschicht z.B. beim Einsatz als wirkstofffreies Wärmepflaster therapeutisch genutzt werden. Hiermit können beispielsweise rheumatische Beschwerden, wie z.B. Ischias, Hexenschuss, Nackensteifigkeit, Schulter-Arm-Schmerzen etc. behandelt werden.

Nach dem Abschalten und gegebenenfalls dem Abnehmen des Induktionssystems (60) kann das therapeutische System (20) auf dem Arm (1) verbleiben und braucht nicht abgenommen zu werden. Nach einem erneuten Aufsetzen des Induktionssystems (60) kann die Wärme- und/oder die Wirkstoffzufuhr wieder erhöht werden. So kann beispielsweise bei Bedarf mehrmals täglich eine Wärmetherapie bzw. bei wirkstoffhaltigen Systemen eine zeitbegrenzte Dosiserhöhung erfolgen. Die Temperatur ist hierbei individuell steuerbar.

Sind beispielsweise am Körper mehrere therapeutische Systeme (20) angeordnet, kann an diese zeitlich nacheinander dasselbe Induktionssystem (60) angeschlossen werden. Auch ist es denkbar, an ein therapeutisches System (20) unterschiedlich aufgebaute Induktionssysteme (60) anzuschließen. Hiermit können z.B. je nach Erfordernis unterschiedliche Zonen des therapeutischen Systems (20) angesprochen werden. Auch ist es denkbar, die Therapievorrichtung (10) mit unterschiedlichen Energiestufen zu betreiben.

Die Figur 4 zeigt eine Therapievorrichtung (10) mit zwei Induktionssystemen (30, 60). Ein erstes, vom therapeutischen System (20) abnehmbares Induktionssystem (60) ist kappenartig auf das therapeutische System (20) aufgesetzt. Dieses Induktionssystem (60) umfasst in diesem Ausführungsbeispiel drei Spulen (73 - 75). Eine erste Spule (73) mit drei spiralförmig angeordneten Windungen ist beispielsweise oberhalb des therapeutischen Systems (20) angeordnet. Die anderen beiden Spulen (74, 75) sind in diesem Ausführungsbeispiel um die Stirnflächen des therapeutischen Systems (20) herum z.B. übereinander angeordnet. Hierbei ist z.B. eine obere Induktionsspule (74) um die obere Laminatschicht (22) und eine untere Induktionsspule (75) um die adhäsive Schicht (21) herum angeordnet. Mittels dieser Spulen (73 - 75) können beispielsweise einzelne Laminatschichten (21, 22) gezielt angesprochen werden, um z.B. die Wirkstoffdiffusion in einer einzelnen Schicht (21, 22) zu erhöhen.

Das zweite Induktionssystem (30) ist in das therapeutische System (20) integriert. In diesem Ausführungsbeispiel umfasst dieses Induktionssystem (30) eine zentral angeordnete Spule (31). Die Spulenquerschnittsfläche ist beispielsweise in der Darstellung der Figur 4 parallel zur Haut (2) angeordnet. Die Spule (31) ist elektrisch z.B. mittels einer hier nicht dargestellten federbelasteten Kontaktverbindung mit dem ersten Induktionssystem (60) verbunden. Beispielsweise kann sie mittels eines Schalters zu- oder abgeschaltet werden. Bei einer Einbindung in den Stromkreis kann sie einzeln oder parallel mit einer oder mehreren anderen Spulen (73 - 75) betrieben werden.

Ist bei eingeschaltetem erstem Induktionssystem (60) die Spule (31) des zweiten Induktionssystems (30) abgeschaltet oder elektrisch getrennt, beeinflusst das vom ersten Induktionssystem (60) aufgebaute Magnetfeld das zweite Induktionssystem (30). Das zeitlich veränderliche Magnetfeld des ersten Induktionssystems (60) durchdringt die in das therapeutische System (20) integrierte Spule (31). In dieser Spule (31) werden Wechselspannungen induziert. Hierdurch gibt die Spule Wärme ab. Die Umgebung der Spule (31) wird aufgewärmt. Sie trägt nun gemeinsam mit den superparamagnetischen Nanopartikeln (40) zur Veränderung der Wirkstoffdiffusion und/oder zur Temperaturerhöhung zu therapeutischen Zwecken bei.

In dem in der Figur 4 dargestellten Ausführungsbeispiel sind zwei Wirkstoffe (23, 24) in unterschiedlicher Konzentration eingelagert. Nach der z.B. getrennten Herstellung in einer wirkstoffhaltigen Laminatschicht (22) und in einer adhäsiven Laminatschicht (21) hat sich die Konzentration entsprechend der Löslichkeit in den einzelnen Schichten (21, 22) eingestellt. In beiden Laminatschichten (21, 22) sind superparamagnetische Nanopartikel (40) in unterschiedlicher Konzentration eingebettet. Gegebenenfalls können die superparamagnetischen Nanopartikel (40) auch nur in einer der beiden Schichten (21, 22) eingelagert sein.

Mittels der beiden Induktionssysteme (60, 30) kann die Wirkstoffabgabe des therapeutischen Systems (20), z.B. bei zum Teil ungelöst vorliegendem Wirkstoff (23, 24) gezielt gesteuert werden. So kann das Konzentrationsverhältnis der Wirkstoffe (23, 24) zueinander verändert werden oder die Diffusionsrate eines der Wirkstoffe (23, 24) erhöht werden.

Liegt beispielsweise einer der Wirkstoffe (23; 24) oder liegen beide Wirkstoffe (23, 24) in weitgehend ungelöster Form vor, steht der nichtgelöste Anteil oder stehen die nichtgelösten Anteile für einen Konzentrationsausgleich und für eine passive Diffusion nicht zur Verfügung. Die superparamagnetischen Nanopartikel (40) sind entweder in einer der beiden Laminatschichten (21; 22) oder in beiden Laminatschichten (21, 22) z.B. in unterschiedlicher Konzentration angeordnet.

Nach dem Aufkleben des transdermalen therapeutischen Systems (20) auf die Haut wird z.B. nur der gelöst vorliegende erste Wirkstoff an die Haut abgegeben.

Nach dem Einschalten des elektrisch ansteuerbaren Systems (50) führt die Temperaturerhöhung, z.B. auf Temperaturen weit oberhalb der Körpertemperatur, zu einem Auflösen des zunächst ungelöst vorliegenden zweiten Wirkstoffs. Dieser Wirkstoff steht nun ebenfalls für die Diffusion zur Verfügung.

Der Konzentrationsausgleich zwischen beiden Laminatschichten (21, 22) kann auch mittels einer, z.B. als Zwischenschicht ausgebildeten beispielsweise semipermeablen Steuermembran beeinflusst werden. In dieser Steuermembran können, zusätzlich oder alternativ zu den Laminatschichten (21, 22), superparamagnetische Nanopartikel (40) eingelagert sein.

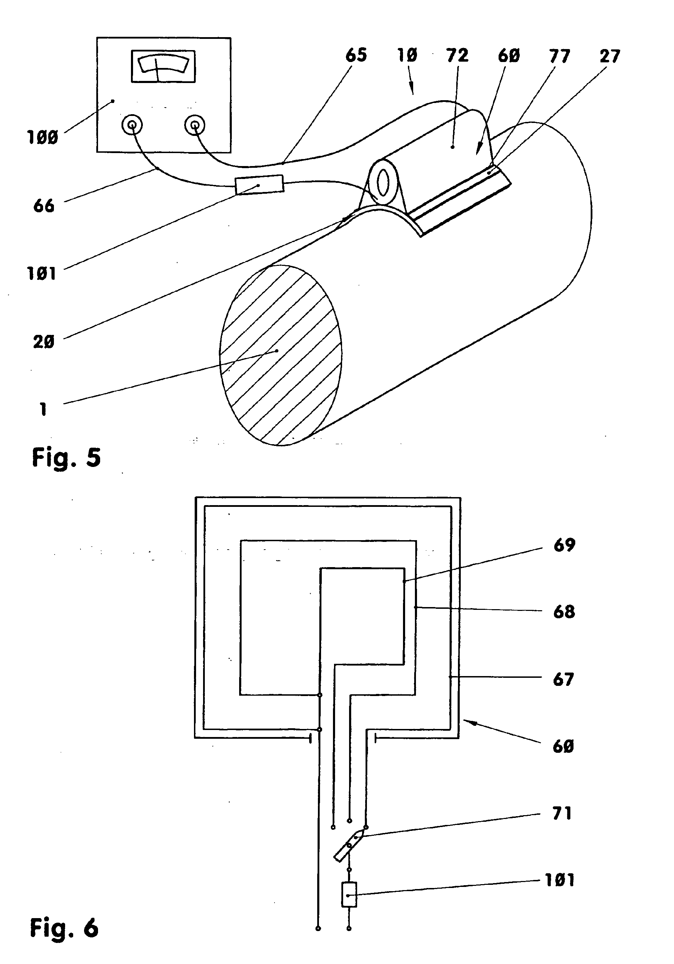

In der Figur 5 ist eine Therapievorrichtung (10) dargestellt, deren Induktionssystem (60) eine zylindrische Spule (72) umfasst. Diese Langspule (72) hat beispielsweise zweihundert Windungen. Die magnetische Neuausrichtung der einzelnen superparamagnetischen Nanopartikel (40) erfolgt aufgrund der schnellen Änderung der magnetischen Flußdichte quasi schlagartig. Die superparamagnetischen Nanopartikel (40) können damit eine hohe Energie freisetzen. Das Induktionssystem (60) hat z.B. seitliche Schienen (77), mit denen es in Einsteckführungen (27) des therapeutischen Systems (20) sitzt und an einen nicht dargestellten Anschlag anliegt. Auch die zylindrische Spule (72) kann einen Spulenkern aufweisen.

In der Figur 6 ist ein Induktionssystem (60) mit z.B. drei Induktionsspulen (67 - 69) und mit einem Umschalter (71) dargestellt. Die Spulen (67 - 69) sind beispielsweise ineinander verschachtelt angeordnet und haben z.B. unterschiedliche Querschnittsflächen. Die Querschnittsfläche der äußeren Spule (67) entspricht beispielsweise der Größe der Oberfläche des therapeutischen Systems (20). Beim Betrieb dieser Induktionsspule (67) werden beispielsweise alle im therapeutischen System (20) angeordneten superparamagnetischen Nanopartikel (40) angesprochen.

Die mittlere Spule (68) liegt innerhalb dieser äußeren Spule (67). Ihre Querschnittsfläche beträgt beispielsweise die Hälfte der Querschnittsfläche der äußeren Spule. Mittels dieser Spule (68) kann der Energieeintrag auf den zentralen Bereich des therapeutischen Systems (20) konzentriert werden.

Die innere Spule (69) liegt in der Draufsicht der Figur 6 z.B. im rechten Bereich der mittleren Spule (68). Ihre Querschnittsfläche beträgt beispielsweise 15 % der Querschnittsfläche der äußeren Spule (67). Wird diese Spule (69) elektrisch angesteuert, werden die superparamagnetischen Nanopartikel (40) vor allem im rechten Bereich des therapeutischen Systems (20) angeregt. In diesem Bereich erfolgt dann die Wärmeentwicklung, die die Wirkstoffdiffusion beschleunigt oder die Wärmetherapie ermöglicht.

Die einzelnen dargestellten Spulen (67 - 69) können jeweils mehr als eine Windung aufweisen. Auch der Einsatz eines oder mehrerer Spulenkerne ist denkbar.

Ist beispielsweise im rechten Bereich des therapeutischen Systems (20) die Konzentration der superparamagnetischen Nanopartikel (40) höher als in den anderen Bereichen des therapeutischen Systems (20), ist mittels der dargestellten innenliegenden Spule (69) z.B. kurzfristig eine konzentrierte Wärmezufuhr möglich. Das therapeutische System (20) kann hierfür in diesem rechten Bereich eine separate Laminatschicht umfassen.

Es ist auch denkbar, in dem in der Figur 6 dargestellten Induktionssystem (60) zwei (67, 68; 67, 69; 68, 69) oder alle Spulen (67 - 69) gleichzeitig anzusteuern. Die magnetischen Felder der Spulen (67 - 69) beeinflussen sich gegenseitig. In einigen Bereichen wird das magnetische Feld des gesamten Induktionssystems (60) verstärkt, in anderen abgeschwächt. Auch ist es denkbar, gleichzeitig jede Spule (67; 68; 69) einzeln z.B. mit unterschiedlicher Frequenz anzusteuern. Aufgrund der sich ergebenden Schwebungen und Resonanzen der Erregerfrequenzen schwankt die Temperatur der einzelnen Bereiche des therapeutischen Systems (20) während der Behandlungszeit. Gegebenenfalls kann so z.B. ein umlaufendes oder springendes Temperaturmaximum erzeugt werden.

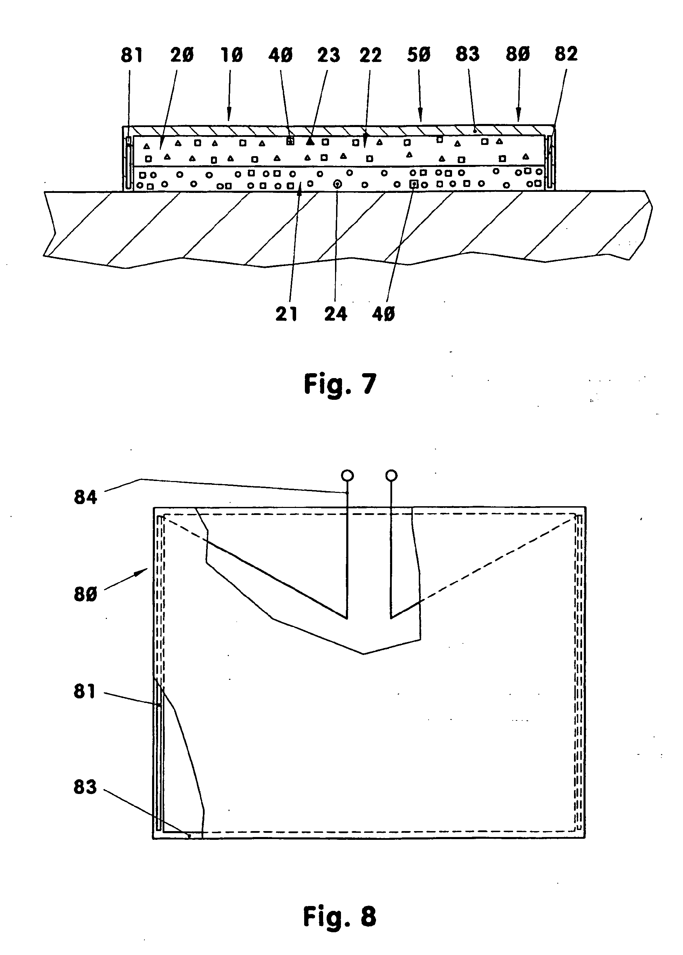

In der Figur 7 ist eine Therapievorrichtung (10) mit einem therapeutischen System (20) und mit einem elektrisch ansteuerbaren System (50) in der Bauart eines kapazitiven Systems (80) dargestellt.

Das therapeutische System (20) ist beispielsweise so aufgebaut wie im Zusammenhang mit den Figuren 1 bis 6 beschrieben. Der Wirkstoff (23) und die superparamagnetischen Nanopartikel (40) sind sowohl in der oberen Laminatschicht (22) als auch in der adhäsiven Laminatschicht (21) in unterschiedlicher, über die Schnittebene der Figur 7 nicht konstanter Konzentration eingelagert.

Das kapazitive System (80) umfasst z.B. zwei einander gegenüberliegende Kondensatorplatten (81, 82), zwischen denen das therapeutische System (20) angeordnet ist. Das kapazitive System (80) ist beispielsweise mittels einer kraft- und/oder formschlüssigen lösbaren Verbindung, z.B. mittels Saugnäpfen, mit dem therapeutischen System (20) verbunden.

Die Figur 8 zeigt eine Draufsicht auf ein derartiges kapazitives System (80). Die Kondensatorplatten (81, 82) sind in einen Kondensatorträger (83) eingebettet. Mittels der Anschlussleitungen (84) sind sie an eine Wechselstromquelle (100) anschließbar.

In einem in einen Wechselstromkreis eingebundenen kapazitiven System (80) sinkt der elektrische Scheinwiderstand mit zunehmender Frequenz der Wechselstromquelle (100). Die auf die Zeit bezogene Änderungsrate der Ladung der Kondensatorplatten (81, 82) und die auf die Zeit bezogene Änderungsrate der Verschiebungsdichte steigen mit zunehmender Frequenz an.

Nach dem Anschluss des kapazitiven Systems (80) an eine Wechselstromquelle (100) wird zwischen den Kondensatorplatten (81, 82) ein zeitlich veränderliches elektrisches Feld erzeugt. Das therapeutische System (20) mit den eingelagerten superparamagnetischen Nanopartikeln (40) bildet das Dielektrikum. In den superparamagnetischen Nanopartikeln (40) bewirkt das wechselnde elektrische Feld eine wechselnde Polarität. Die superparamagnetischen Nanopartikel (40) geben eine Teil der von ihnen aus dem elektrischen Feld aufgenommenen Energie als Wärme ab. Beispielsweise führt die Temperaturerhöhung des Systems zu erhöhten Diffusionskoeffizienten der Wirkstoffe in den Zubereitungen und somit zu einer verbesserten Wirkstoffabgabe.

Um die von den superparamagnetischen Nanopartikeln (40) abgegebene Wärme weiter zu erhöhen, kann beispielsweise die an das kapazitive System (80) angelegte Spannung erhöht werden. Auch kann zur Veränderung der Wärmeabgabe z.B. im kapazitiven (80) und/oder im therapeutischen System (20) ein zusätzliches Dielektrikum angeordnet werden oder die Frequenz der Wechselstromquelle (100) geändert werden.

Mit dem Abschalten der Wechselstromquelle (100) wird das elektrische Feld abgeschaltet. Die superparamagnetischen Nanopartikel (40) nehmen den für sie energetisch günstigsten Zustand ein. Sie geben keine weitere Wärme ab. Die Diffusionsrate der Wirkstoffe (23, 24) im therapeutischen System (20) sinkt auf ihren ursprünglichen Wert.

Es ist auch denkbar, dass die elektrisch ansteuerbare Vorrichtung (50) der Therapievorrichtung (10) sowohl ein kapazitives (80) als auch eine induktives System (60) umfasst. Diese können beispielsweise alternativ eingeschaltet werden, um z.B. unterschiedliche Zonen des therapeutischen Systems anzusprechen.

Auch Kombinationen der verschiedenen Ausführungsformen sind denkbar.

### Bezugszeichenliste:

- 1: Arm
- 2: Haut

- 10: Therapievorrichtung

- 20: Therapeutisches System, transdermales therapeutisches System
- 21: adhäsive Laminatschicht, Laminatschicht
- 22: obere Laminatschicht, Laminatschicht
- 23: Wirkstoff, erster Wirkstoff. Pharmazeutische Wirkstoffzubereitung
- 24: zweiter Wirkstoff
- 25: Verbundmasse
- 26: Deckfolie
- 27: Einsteckführung

- 30: zweites Induktionssystem
- 31: Spule, innenliegend

- 40: superparamagnetische Nanopartikel

- 50: elektrisch ansteuerbares System

- 60: Induktionssystem
- 61: Umgriffsklammern, Verschlussteil
- 62: Umgriffsbänder, Verschlussteil
- 63: Spulenträger
- 64: Induktionsspule, Spule
- 65, 66: Leitungen
- 67: äußere Induktionsspule
- 68: mittlere Induktionsspule
- 69: innere Induktionsspule

- 71: Umschalter
- 72: zylindrische Spule, Langspule
- 73: Induktionsspule
- 74: Induktionsspule, oben
- 75: Induktionsspule, unten
- 76: Haftverschluss
- 77: Schiene

- 80: kapazitives System
- 81: Kondensatorplatte
- 82: Kondensatorplatte
- 83: Kondensatorträger
- 84: Anschlussleitungen

- 100: Wechselstromquelle
- 101: Vorwiderstand

## Patentansprüche

1. Therapeutisches System (20), das laminatförmig aufgebaut ist und das zumindest eine adhäsive Laminatschicht (21) umfasst, **dadurch gekennzeichnet,**
**dass** zumindest eine Laminatschicht (21; 22) und/oder eine zwischen zwei Laminatschichten (21, 22) angeordnete Zwischenschicht superparamagnetische Nanopartikel (40) enthält.

2. Therapeutisches System (20) nach Anspruch 1, **dadurch gekennzeichnet, dass** die superparamagnetischen Nanopartikel (40) eine maximale Ausdehnung zwischen fünf und dreißig Nanometern haben.

3. Therapeutisches System (20) nach Anspruch 1, **dadurch gekennzeichnet, dass** die Masse aller in eine Laminatschicht (21; 22) eingelagerten superparamagnetischen Nanopartikel (40) zwischen 0,5 Prozent und 25 Prozent der Masse dieser Laminatschicht (21; 22) beträgt.

4. Therapeutisches System (20) nach Anspruch 1, **dadurch gekennzeichnet, dass** es eine wirkstoffhaltige Schicht (22) enthält.

5. Therapeutisches System (20) nach Anspruch 4, **dadurch gekennzeichnet, dass** in der wirkstoffhaltigen Schicht (22) superparamagnetischen Nanopartikel (40) eingebettet sind.

6. Therapeutisches System (20) nach Anspruch 1, **dadurch gekennzeichnet, dass** es ein integriertes Induktionssystem (30) umfasst.

7. Therapievorrichtung (10) mit einem therapeutischen System (20) nach einem der Ansprüche 1 - 6 und mit mindestens einem lösbar mit dem therapeutischen System (20) verbundenem elektrisch ansteuerbaren System (50),
- wobei das elektrisch ansteuerbare System (50) einen frequenzabhängigen elektrischen Scheinwiderstand aufweist und
- wobei beim Betrieb der Therapievorrichtung (10) das elektrisch ansteuerbare System (50) ein in der Ausrichtung und der Stärke zeitlich veränderliches, die superparamagnetischen Nanopartikel (40) durchdringendes elektrisches oder magnetisches Feld erzeugt.

8. Therapievorrichtung (10) nach Anspruch 7, **dadurch gekennzeichnet, dass** das elektrisch ansteuerbare System (50) ein Induktionssystem (60) ist.

## Claims

1. Therapeutic system (20), which has a laminate structure and comprises at least one adhesive laminate layer (21), **characterized in that** at least one laminate layer (21; 22) and/or an intermediate layer arranged between two laminate layers (21, 22) contains superparamagnetic nanoparticles (40).

2. Therapeutic system (20) according to Claim 1, **characterized in that** the superparamagnetic nanoparticles (40) have a maximum extent of between five and thirty nanometres.

3. Therapeutic system (20) according to Claim 1, **characterized in that** the mass of all the superparamagnetic nanoparticles (40) incorporated in a laminate layer (21; 22) is between 0.5 per cent and 25 per cent of the mass of this laminate layer (21; 22).

4. Therapeutic system (20) according to Claim 1, **characterized in that** it comprises a layer (22) containing active substance.

5. Therapeutic system (20) according to Claim 4, **characterized in that** superparamagnetic nanoparticles (40) are embedded in the layer (22) containing active substance.

6. Therapeutic system (20) according to Claim 1, **characterized in that** it comprises an integrated induction system (30).

7. Therapy device (10) with a therapeutic system (20) according to one of Claims 1 to 6, and with at least one electrically actuatable system (50) releasably connected to the therapeutic system (20),
- wherein the electrically actuatable system (50) has a frequency-dependent electrical impedance, and
- wherein, during the operation of the therapy device (10), the electrically actuatable system (50) generates an electric or magnetic field which varies in orientation and strength over time and which penetrates the superparamagnetic nanoparticles (40).

8. Therapy device (10) according to Claim 7, **characterized in that** the electrically actuatable system (50) is an induction system (60).

## Revendications

1. Système thérapeutique (20) qui présente une forme stratifiée et qui comprend au moins une couche stratifiée adhésive (21), **caractérisé en ce qu'**au moins une couche stratifiée (21 ; 22) et/ou une couche intermédiaire disposée entre deux couches stratifiées (21, 22) contien(nen)t des nanoparticules superparamagnétiques (40).

2. Système thérapeutique (20) selon la revendication 1, **caractérisé en ce que** les nanoparticules superparamagnétiques (40) présentent une dimension maximale entre 5 et 30 nanomètres.

3. Système thérapeutique (20) selon la revendication 1, **caractérisé en ce que** la masse de toutes les nanoparticules superparamagnétiques (40) incorporées dans une couche stratifiée (21 ; 22) représente entre 0,5% et 25% de la masse de cette couche stratifiée (21 ; 22).

4. Système thérapeutique (20) selon la revendication 1, **caractérisé en ce qu'**il contient une couche (22) contenant une substance active.

5. Système thérapeutique (20) selon la revendication 4, **caractérisé en ce que** des nanoparticules superparamagnétiques (40) sont incorporées dans la couche (22) contenant une substance active.

6. Système thérapeutique (20) selon la revendication 1, **caractérisé en ce qu'**il comprend un système d'induction (30) intégré.

7. Dispositif thérapeutique (10) présentant un système thérapeutique (20) selon l'une quelconque des revendications 1-6 et pourvu d'au moins un système (50) pouvant être commandé électriquement, relié de manière amovible au système thérapeutique (20),
- le système (50) pouvant être commandé électriquement présentant une impédance électrique dépendant de la fréquence et
- le système (50) pouvant être commandé électriquement, lors du fonctionnement du dispositif thérapeutique (10), générant un champ électrique ou magnétique à direction et à intensité modifiables dans le temps, pénétrant dans les nanoparticules superparamagnétiques (40).

8. Dispositif thérapeutique (10) selon la revendication 7, **caractérisé en ce que** le système (50) pouvant être commandé électriquement est un système à induction (60).
